# EUROPEAN PATENT APPLICATION

(11) **EP 2 998 283 A1**
(43) Date of publication of application: **23.03.2016**
(21) Application number: 15185535.0
(22) Date of filing: 16.09.2015
(51) Int. Cl.: C07C 41/16, C07C 43/12

(54) **1,2-BIS(2,2-DIFLUOROETHOXY) ETHANE AND MANUFACTURING METHOD THEREOF**

(30) Priority: 16.09.2014 JP 2014187747
(71) Applicant: HONDA MOTOR CO., LTD., Tokyo, 107-8556 (JP); TOSOH F-TECH, INC., Shunan Yamaguchi 746-0006 (JP)
(72) Inventor: CHIBA, Mahito, Wako-shi Saitama 351-0193 (JP)
(74) Representative: Michalski Hüttermann & Partner Patentanwälte mbB

(57) **Abstract**

1,2-Bis(2,2-difluoroethoxy)ethane which can be used as electrolyte solution of a non-aqueous electrolyte secondary battery is provided. Used are 1,2-Bis(2,2-difluoroethoxy)ethane represented by the following formula (1) : 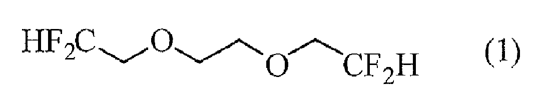 and a method for manufacturing 1,2-bis(2,2-difluoroethoxy)ethane including the step of reacting 2-(2,2-difluoroethoxy)ethanol represented by the following formula (2) with a compound represented by the general formula (3) in a basic compound or a basic compound-containing solvent: 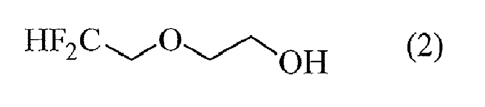 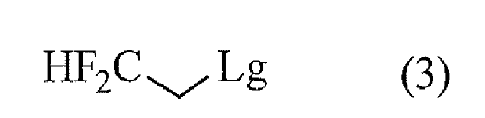 wherein Lg represents an anionic leaving group.

## Description

### BACKGROUND OF THE INVENTION

### Field of the Invention

The present invention relates to 1,2-bis(2,2-difluoroethoxy)ethane which can be used as electrolyte solution of a non-aqueous electrolyte secondary battery.

### Description of the Related Art

A non-aqueous electrolyte secondary battery has a higher voltage and a higher energy density in comparison with a secondary battery including an aqueous electrolyte solution. A non-aqueous solvent including fluorinated dialkoxyethane is effective for improving the power output and the oxidation resistance, due to having a small viscosity. Among the solvent, CH₃OCH₂CH₂OCH₂CF₃ is supposed to be a suitable non-aqueous solvent, having a low viscosity and a high relative permittivity (see, for example, Japanese Patent Laid-Open No. 1-117838). Further, physical properties of CH₃OCH₂CH₂OCH₂CF₃ and CH₃CH₂OCH₂CH₂OCH₂CF₃ relating to properties as electrolyte solution are described (see, for example, G. Shimazaki et al., "Physical properties of trifluorodialkoxyethane and properties of electrolyte solution", Proceedings of the 73rd Meeting of the Electrochemical Society of Japan (Apr. 1, 2006), p. 242).

Since the presence of halide ions such as chlorine ions in electrolyte solution of a lithium ion secondary battery usually causes substantial degradation of the battery properties, the halide ion content is desired to be as low as possible.

In general, a fluorine-containing alkoxy metal salt and an ether compound substituted with a leaving group are used for synthesizing a fluorine-containing glyme compound which can be used as the electrolyte solution of a non-aqueous electrolyte battery (see, for example, Japanese Patent Laid-Open No. 2010-241772). In the case of a halogen leaving group, the halide ions are contained in the reaction residue. It is therefore feared that the halide ions remain in the isolated and purified fluorine-containing glyme compound.

### SUMMARY OF THE INVENTION

Although various fluorinated glymes are described as above, any known compound having a 2,2-difluoroethoxy group (CF₂HCH₂O-) has the functional group on one terminal only. In contrast, 1,2-bis(2,2-difluoroethoxy) ethane (CF₂HCH₂OCH₂CH₂OCH₂CF₂H) of the present invention, which has 2,2-difluoroethoxy groups on both terminals, is a novel compound which has not been manufactured before.

In synthesis of the novel compound of the present invention, for example, for use as electrolyte solution, the use of a compound substituted with a halogen is required to be avoided to the utmost in order to prevent reduction in battery properties.

Through extensive investigation, the present inventors have found 1,2-bis(2,2-difluoroethoxy)ethane which can be used as electrolyte solution of a non-aqueous electrolyte secondary battery, and adopted a manufacturing method using no compound substituted with a halogen , so that the present invention has been accomplished.

More specifically, the present invention relates to 1,2-bis(2,2-difluoroethoxy)ethane represented by the following formula (1) : 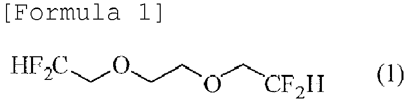 and further relates to a method for manufacturing 1,2-bis(2,2-difluoroethoxy)ethane including reacting 2-(2,2-difluoroethoxy)ethanol represented by the following formula (2) : 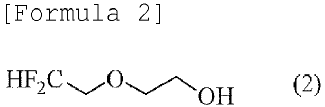 with a compound represented by the following general formula (3): 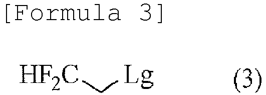 wherein Lg represents an anionic leaving group.

Through extensive investigation of the synthesis of 1,2-bis(2,2-difluoroethoxy)ethane of the present invention, it was found that 1,2-bis(2,2-difluoroethoxy)ethane having a less final content of halide ions can be manufactured through incorporation of the step of synthesizing 2-(2,2-difluoroethoxy)ethanol having an extended oxyethylene chain by using ethylene carbonate, thereby accomplishing the present invention.

According to the present invention, a novel compound 1,2-bis(2,2-difluoroethoxy)ethane which can be used as an electrolyte solution of non-aqueous electrolyte secondary battery can be synthesized and provided. Further, according to the manufacturing method of the present invention, 1,2-bis(2,2-difluoroethoxy)ethane can be synthesized to have a halide ion content of less than 10 ppm by weight, which is industrially useful in application to an electrolyte solution and the like.

### DETAILED DESCRIPTION OF THE PREFERRED EMBODIMENTS

The present invention is described in more detail in the following.

1,2-Bis(2,2-difluoroethoxy)ethane of the present invention is represented by the following formula (1):

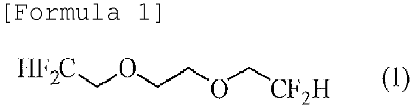

1,2-Bis(2,2-difluoroethoxy)ethane can be synthesized by reacting 2-(2,2-difluoroethoxy)ethanol represented by the following formula (2): 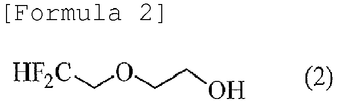 with a compound represented by the following general formula (3): 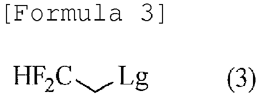 in a basic compound or a basic compound-containing solvent, wherein Lg represents an anionic leaving group.

In manufacturing 1,2-bis(2,2-difluoroethoxy)ethane of the present invention, the anionic leaving group (referred to as Lg) is preferably other than halogens. Any reactive group may be used, and particularly preferred examples include a p-toluene sulfonyloxy group (p-CH₃C₆H₄SO₃-) and a trifluoromethane sulfonyloxy group (CF₃SO₃-). The amount used of a compound represented by the formula (3) may be 1.0 times or more, preferably 1.1 or more times to 2.0 times or less, the moles of 2-(2,2-difluoroethoxy)ethanol for use in the reaction.

Examples of the basic compound for use in manufacturing 1,2-bis(2,2-difluoroethoxy)ethane of the present invention include lithium hydride, sodium hydride, potassium hydride, calcium hydride, metallic sodium, butyl lithium, lithium diisopropylamide, lithium hydroxide, sodium hydroxide, potassium hydroxide, calcium hydroxide, sodium carbonate, potassium carbonate, triethylamine, pyridine, picoline, lutidine and sodium amide. In the case of a basic compound creating water as byproduct in forming an alkoxy salt, reduction in yield is feared. Accordingly, lithium hydride, sodium hydride, potassium hydride, calcium hydride, butyl lithium, and lithium diisopropylamide may be preferably used in an amount of 1.0 times or more, preferably 1.1 times or more to 2.0 times or less, the moles of 2-(2,2-difluoroethoxy)ethanol for use in the reaction.

In manufacturing 1,2-bis(2,2-difluoroethoxy)ethane of the present invention, examples of the solvent for use in the reaction include diethyl ether, methyl tertiary butyl ether, tetrahydrofuran, diisopropyl ether, di-n-butyl ether, dioxane, dioxolane, dimethyl sulfoxide, acetonitrile, pyridine, picoline, lutidine, and collidine, which may be used in an amount of 2.0 parts or more to 40.0 parts or less, preferably 5.0 parts or more to 15.0 parts or less relative to 2-(2,2-difluoroethoxy)ethanol for use in the reaction.

In the reaction for manufacturing 1,2-bis(2,2-difluoroethoxy)ethane of the present invention, 2-(2,2-difluoroethoxy)ethanol represented by the formula (2) may be dripped into a mixture previously prepared from a basic compound, a solvent, and a compound represented by the formula (3), or the dripping sequence of 2-(2,2-difluoroethoxy)ethanol represented by the formula (2) and the compound represented by the formula (3) may be inverted. The reaction may be performed at -20°C or more to 50°C or less, preferably in the range from 5°C or more to 30°C or less, and more preferably in the range from the melting point or more of a solvent for use to 30°C in the case of the solvent with a high melting point.

In manufacturing of 1,2-bis(2,2-difluoroethoxy)ethane of the present invention, the synthesis of 2-(2,2-difluoroethoxy)ethanol represented by the formula (2)is performed preferably without using a compound substituted with a halogen. More specifically, 2-(2,2-difluoroethoxy)ethanol can be obtained by heating 2,2-difluoroethanol and ethylene carbonate in the presence of a basic compound.

Ethylene carbonate for use in the reaction of 2,2-difluoroethanol and ethylene carbonate may be used in an amount of 0.1 times or more the moles of 2,2-difluoroethanol for use in the reaction. With an amount of less than 0.05 times the moles, the reaction time may be extended in some cases, which is inefficient. With an amount of more than 2.0 times the moles, a large amount of remaining ethylene carbonate may cause difficulty in purification procedure in some cases. Accordingly the amount used may be preferably 0.2 times or more the moles to 1.2 times or less the moles.

Examples of the basic compound for use in the reaction of 2,2-difluoroethanol and ethylene carbonate include trimethylamine, triethylamine, triisopropylamine, tributylamine, sodium carbonate, potassium carbonate, and cesium carbonate, though not particularly limited thereto.

The reaction solvent for the reaction of 2,2-difluoroethanol and ethylene carbonate is used on an as needed basis, though the reaction may be performed without using the solvent. In the case of using a reaction solvent, examples of the reaction solvent include hexane, heptane, toluene, benzene, cumene, and xylene, though not particularly limited thereto. The amount of the reaction solvent is 10 parts or less, more preferably 1 part or less, relative to 2,2-difluoroethanol for use in the reaction. Alternatively, in the case of using an amine such as trimethylamine and triethylamine as base, the amine may be used as a substitute for solvent.

The reaction temperature for the reaction of 2,2-difluoroethanol and ethylene carbonate is preferably from 80°C to the boiling point or less of a solvent for use. In the case of using an organic base as the base, the reaction is performed preferably at the boiling point thereof or less. In the case of using a solvent having a boiling point less than 80°C under normal pressure, the reaction is preferably performed at 80°C or more under pressure.

In the post-processing after the reaction for obtaining 1,2-bis(2,2-difluoroethoxy)ethane of the present invention, purification can be performed by concentration and distillation after stopping the reaction with water in a necessary quantity. The amount of water may be the difference in moles between a basic compound and 2-(2,2-difluoroethoxy)ethanol for use in the reaction. After the reaction of 2,2-difluoroethanol with ethylene carbonate, concentration or distillation is directly performed, so that 2-(2,2-difluoroethoxy)ethanol can be purified. Alternatively 2-(2,2-difluoroethoxy)ethanol may be directly used in the subsequent process without purification.

Examples of the halide ions which may possibly be contained in 1,2-bis(2,2-difluoroethoxy)ethane of the present invention include chlorine ions, bromine ions and iodine ions. The content of these halide ions may be simply and accurately measured by a known method such as potentiometric titration.

The content of halide ions (total amount of chlorine ions, bromine ions and iodine ions) in the 1,2-bis(2,2-difluoroethoxy)ethane of the present invention is preferably less than 10 ppm. With a content of halide ions of 10 ppm or more, an adverse effect on the battery performance, for example, degradation of the battery properties, may be brought in repeated charging/discharging of the battery in some cases. Though the reason is not clear, phenomena such as the elution of electrode active material and the progress of corrosion of Al or the like for use as current collector of an electrode in the presence of halide ions may be possibly involved.

### Example

In the following, synthesis of 1,2-bis(2,2-difluoroethoxy)ethane of the present invention will be more specifically described with reference to examples, but the present invention is not limited to the examples. In the analysis, the following instruments were used.
¹H-NMR and ¹⁹F-NMR: AVANCE II 400 manufactured by Bruker Corporation;
GC-MS: GCMS-QP2010 Plus manufactured by SHIMADZU CORPORATION; and
Potentiometric titration: Automatic potentiometric titrator AT-610 manufactured by Kyoto Electronics Manufacturing Co., Ltd.

### <Example 1>

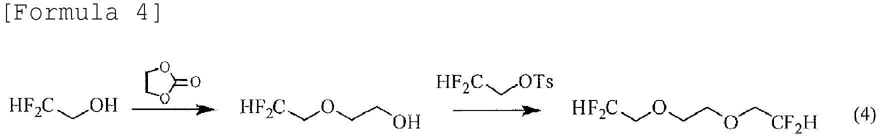

A 200-ml three neck flask having a stirring bar was charged with 3 agents including 2,2-difluoroethanol (34.0 g, 0.41 mol), ethylene carbonate (54.8 g, 0.62 mol), and triethylamine (42.0 g, 0.41 mol), and the mixture was stirred at 95°C for 24 hours after substitution with nitrogen. The reaction liquid was cooled to room temperature and the reaction yield of 2-(2,2-difluoroethoxy)ethanol was determined to be 70% by ¹H-NMR. By a subsequent conventional purification procedure, 2-(2,2-difluoroethoxy)ethanol (31.5 g, 0.25 mol) was obtained.

Subsequently, a 2000-ml three neck flask having a stirring bar was charged with 60% sodium hydride (21.2 g, 0.53 mol), dimethyl sulfoxide (375.2 g), and 2,2-difluoroethyl tosylate (100.0 g, 0.42 mol, HF₂CCH₂OTs in the formula (4)) under nitrogen stream. The mixture was cooled to 10°C or less, and 2-(2,2-difluoroethoxy)ethanol (31.5 g, 0.25 mol) was then slowly dripped therein. The mixture was then stirred at room temperature for 16 hours and quenched with addition of water after reaction. The reaction yield of 1,2-bis(2,2-difluoroethoxy)ethane was determined to be 72% by ¹⁹F-NMR of the quenched mixture. Through a subsequent conventional purification procedure, 30.4 g of 1,2-bis(2,2-difluoroethoxy)ethane was obtained (yield: 64%). Analytical results were as follows. In the NMR analysis, a TMS-containing solvent for measurement was used, and the chemical shift in ¹H-NMR was corrected. In ¹⁹F-NMR, hexafluorobenzene (-162 ppm) was used as internal standard for correction of the chemical shift.
¹H-NMR (MeOD-d4, 400 MHz): 3.69 ppm (dt, 4H, CH₂), 3.71 ppm (s, 4H, OCH₂CH₂O), 5.85 ppm (tt, 2H, CHF₂);
¹⁹F-NMR (MeOD-d4, 400 MHz): -126.1 ppm (dt, 4F, CHF₂);
Boiling point (b. p.): 62-63°C (10 mmHg);
GC-MS (m/z): 170 (m-20, 5), 139 (9), 109 (50), 95 (79), 65 (100), 45 (51), 43 (23), 31 (19).

In GC-MS, though no molecular ion peak was confirmed, de-HF (m-20) was observed. The content of halide ions was determined to be 0.3 ppm (detection limit: 0.1 ppm) by potentiometric titration.

### <Example 2>

The dripping sequence of 2-(2,2-difluoroethoxy)ethanol and 2,2-difluoroethyl tosylate in Example 1 was inverted in implementation. As a result, the reaction yield of 1,2-bis(2,2-difluoroethoxy)ethane was 65%. The yield of 2-(2,2-difluoroethoxy)ethanol as precursor compound was 70%, similarly as in Example 1. The content of halide ions was determined to be 0.4 ppm (detection limit: 0.1 ppm) by potentiometric titration.

### <Examples 3 to 5>

Examples of synthesis of 1,2-bis(2,2-difluoroethoxy)ethane performed in the same way as in Example 1, except only that the basic compound in Example 1 for converting 2-(2,2-difluoroethoxy)ethanol into a metal salt was changed, are described in the following. The detection limit of the content of halide ions was 0.1 ppm.

**[Table 1]**

| | Basic compound | Yield | Halide ion content |
|---|---|---|---|
| Example 3 | Potassium hydride (30 wt% in mineral oil) | 69% | 0.3 ppm |
| Example 4 | n-Butyl lithium (1.6 M n-hexane solution) | 51% | 0.7 ppm |
| Example 5 | Potassium hydroxide | 39% | 0.9 ppm |

### [Example 6]

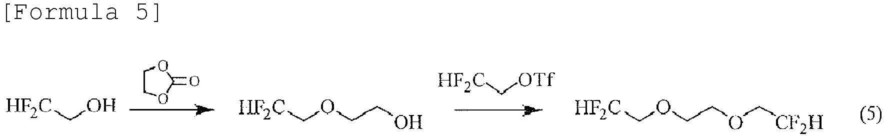

Synthesis of 1,2-bis(2,2-difluoroethoxy)ethane was performed in the same way as in Example 1, except that 2,2-difluoroethyl triflate (HF₂CCH₂OTf in the formula (5)) was used instead of 2,2-difluoroethyl tosylate in Example 1. The reaction yield of 1,2-bis(2,2-difluoroethoxy)ethane was determined to be 70% by ¹⁹F-NMR after reaction. The content of halide ions was determined to be 0.5 ppm (detection limit: 0.1 ppm) by potentiometric titration.

### <Comparative Example 1>

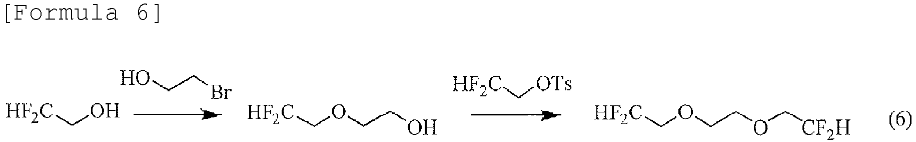

A 100-ml three neck flask having a stirring bar was charged with 60% sodium hydride (4.2 g, 0.11 mol) and dimethylsulfoxide (75.0 g) under nitrogen stream. The mixture was cooled to 10°C or less, and 2,2-difluoroethanol (9.0 g, 0.11 mol) was then slowly dripped therein. The mixture was stirred at 10°C or less for 2 hours and 2-bromoethanol (13.7 g, 0.11 mol) was then slowly dripped therein. The mixture was stirred at room temperature for 4 hours and quenched with addition of water after reaction. The reaction yield of 2-(2,2-difluoroethoxy)ethanol was determined to be 60% by ¹⁹F-NMR of the mixture. Through a subsequent conventional purification procedure, 2-(2,2-difluoroethoxy)ethanol was obtained (6.6 g, 0.052 mol, isolated yield: 47%).

Subsequently, 1,2-bis(2,2-difluoroethoxy)ethane was synthesized in the same way as in Example 1, using the obtained 2-(2,2-difluoroethoxy)ethanol, with a reaction yield of 68% and an isolated yield of 58%. The content of halide ions was determined to be 70.3 ppm (detection limit: 0.1 ppm) by potentiometric titration.

According to the present invention, a novel compound 1,2-bis(2,2-difluoroethoxy)ethane becomes available. Further, according to the manufacturing method of the present invention, a compound having a low halogen content can be produced, so that 1,2-bis(2,2-difluoroethoxy)ethane which can be used as electrolyte solution of a non-aqueous electrolyte secondary battery becomes available.

## Claims

1. 1,2-Bis(2,2-difluoroethoxy)ethane represented by the following formula (1) :

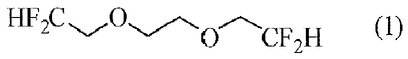

2. 1,2-Bis(2,2-difluoroethoxy)ethane according to claim 1, wherein a content of halide ions (total amount of chlorine ions, bromine ions and iodine ions) is less than 10 ppm by weight.

3. A method for manufacturing 1,2-bis(2,2-difluoroethoxy)ethane according to claim 1 or claim 2 comprising the step of reacting 2-(2,2-difluoroethoxy)ethanol represented by the following formula (2) : 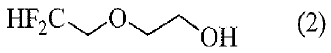 with a compound represented by the general formula (3): 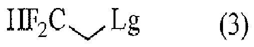 in a basic compound or a basic compound-containing solvent, wherein Lg represents an anionic leaving group.

4. The method for manufacturing 1,2-bis(2,2-difluoroethoxy)ethane according to claim 3, wherein the basic compound comprises at least one selected from the group consisting of lithium hydroxide, sodium hydroxide, potassium hydroxide, calcium hydroxide, lithium hydride, sodium hydride, potassium hydride, calcium hydride, metallic sodium, butyl lithium, lithium diisopropylamide, sodium carbonate, potassium carbonate, triethylamine, pyridine, picoline, and lutidine.

5. The method for manufacturing 1,2-bis(2,2-difluoroethoxy)ethane according to claim 3, wherein the basic compound comprises at least one selected from the group consisting of lithium hydride, sodium hydride, potassium hydride, calcium hydride, butyl lithium, and lithium diisopropylamide.

6. The method for manufacturing 1,2-bis(2,2-difluoroethoxy)ethane according to any one of claims 3 to 5, wherein the leaving group Lg in the general formula (3) is a p-toluene sulfonyloxy group (p-CH₃C₆H₄SO₃-) or a trifluoromethane sulfonyloxy group (CF₃SO₃-).

7. The method for manufacturing 1,2-bis(2,2-difluoroethoxy)ethane according to any of the claims 3 to 6, wherein the synthesis step of 2-(2,2-difluoroethoxy)ethanol represented by the formula (2) comprises reacting 2,2-difluoroethanol with ethylene carbonate.
